**Europäisches Patentamt**

**European Patent Office**

**Office européen des brevets**

(19)

(11) Publication number: **0 298 644**
**A2**

# EUROPEAN PATENT APPLICATION

(12)

(21) Application number: **88305895.0**

(22) Date of filing: **29.06.88**

(51) Int. Cl.⁴: **A61K 31/24 , A61K 31/20 , A61K 31/28**

The title of the invention has been amended (Guidelines for Examination in the EPO, A-III, 7.3).

(30) Priority: **07.07.87 GB 8715914**

(43) Date of publication of application:
**11.01.89 Bulletin 89/02**

(84) Designated Contracting States:
**AT BE CH DE ES FR GB GR IT LI LU NL SE**

(71) Applicant: **EFAMOL HOLDINGS PLC**
**Efamol House Woodbridge Meadows**
**Guildford Surrey GU1 1BA(GB)**

(72) Inventor: **Horrobin, David Frederick**
**c/o Efamol House Woodbridge Meadows**
**Guildford Surrey GU1 1BA(GB)**
Inventor: **Corrigan, Frank**
**c/o Argyll & Bute Hospital**
**Lochgilphead, Argyll PA3T 8ED**
**Scotland(GB)**

(74) Representative: **Caro, William Egerton et al**
**J. MILLER & CO. Lincoln House 296-302 High**
**Holborn**
**London WC1V 7JH(GB)**

(54) **Indium, caesium or cerium compounds for treatment of cerebral disorders.**

(57) A method of treatment of schizophrenia or other chronic cerebral disorder in which cerebral atrophy is shown, is characterised in that one or more assimilable or pharmaceutically acceptable indium caesium or cerium compounds is associated with a pharmaceutically acceptable diluent or carrier.

EP 0 298 644 A2

## TREATMENT OF CEREBRAL DISORDERS

### FIELD OF INVENTION

The invention relates to treatment of cerebral disorders.

### BACKGROUND

Schizophrenia is a disease of unknown aetiology. It is often described as consisting of two sets of symptoms shown as "positive" and "negative". The positive symptoms include bizarre behaviour and hallucinations, while the negative symptoms include social and emotional withdrawal and loss of emotional responsiveness. Drugs which interfere with the activation of dopamine receptors ("dopamine blockers") are effective in the control of positive symptoms and are in practice the only drugs specifically used for the treatment of schizophrenia. Unfortunately these drugs have little or no action on the negative symptoms and may possibly make those symptoms worse. In addition the dopamine blockers can have various side effects, including the serious one of tardive dyskinesia. There is therefore a need for the development of new approaches to schizophrenia.

### NEW OBSERVATIONS

We have recently made observations which suggest a completely new treatment. Many elements are found in the body in almost imperceptible traces and it is generally unknown whether or not these are essential for human function. However, a number of these elements, such as selenium and cobalt, are now known to be required for normal human metabolism. We have therefore carried out a survey of the levels of trace elements in the brains taken from patients who died after having had schizophrenia during life and from control individuals who were not known to have had schizophrenia. We have unexpectedly found that three trace elements, cerium, caesium and indium, occurred in schizophrenic brains at levels which were significantly lower than those in non-schizophrenic controls. The results are shown in Tables 1 and 2.

TABLE 1

| Concentrations of indium and cerium in the frontal cerebral cortex of schizophrenic and non-schizophrenic individuals. Statistical analysis was by the Mann-Whitney test. | | | |
|---|---|---|---|
| | **SCHIZOPHRENIC (14)** | **NON-SCHIZOPHRENIC (8)** | |
| Indium | $0.0061 \pm 0.0012$ | $0.0074 \pm 0.007$ | $p<0.008$ |
| Cerium | $2.33 \pm 0.47$ | $2.71 \pm 0.39$ | $p<0.035$ |
| Figures are mean $\pm$ SD and the units are microg/g of tissue. | | | |

TABLE 2

| Concentrations of caesium in the caudate nucleus of brains from schizophrenic and non-schizophrenic individuals. Units and analysis as in Table 1. | | |
|---|---|---|
| **SCHIZOPHRENIC** | **NON-SCHIZOPHRENIC** | |
| 0.33 ± 0.05 | 0.37 ± 0.05 | p<0.01 |

These results suggest that deficiencies of one or more of indium, caesium and cerium are important in the pathogenesis of schizophrenia. It is thus desirable to ensure that their levels in the brain in schizophrenics, become similar to those in controls.

Further, brain atrophy is known to occur in schizophrenia. It is also known to occur in other chronic cerebral disorders such as senile and presenile dementias (including Alzheimer's disease) and in alcoholism. We therefore believe that deficiencies of one or more of indium, caesium and cerium are involved in these disorders also and that they may be treated in the same way.

## THE INVENTION

We therefore propose that patients with schizophrenia and other chronic cerebral disorders in which brain atrophy is shown are treated with appropriate amounts of cerium, caesium, indium or all three or any two of them. Any biologically compatible cerium, caesium or indium compound may be used and the routes of administration include oral, enteral, parenteral (including directly into the cerebrospinal fluid) and percutaneous.

The invention relates not only to the treatment but to methods of preparation of medicaments for use in such treatments where the indium, caesium or cerium compounds or all three or any two of them are associated with a pharmaceutical carrier or diluent to give such medicaments, and to pharmaceutical preparations as such whether for such treatments or any other therapeutic use, since we believe they are new in themselves.

## ESSENTIAL FATTY ACIDS

There is particular value in using cerium, caesium or indium salts of essential fatty acids, especially of those fatty acids which have undergone 6-desaturation. These compounds are believed to be new per se and are therefore also an aspect of the invention, in themselves. The EFAs make up a very large part of the brain by weight. We have recently measured the levels of the EFAs in schizophrenic patients' red blood cell membranes and have found that amounts of both the n-6 and n-3 EFAs are significantly reduced. We have also found that supplementation with EFAs produces a significant improvement in psychiatric status in hospitalised schizophrenics. The EFA salts of indium, caesium or cerium are therefore particularly desirable, combining the two effects. Moreover, they increase the ability of the cerium, caesium and indium to cross the blood brain barrier since this is readily traversed by the EFAs but possibly not by many cerium, caesium and indium compounds. In any event the cerium, caesium or indium are desirably given along with n-6 acids, n-3 acids, or both even if these fatty acids are not chemically combined with the indium but are in some form of physical admixture or are administered concurrently in appropriate dosage forms.

The main dietary EFAs are linoleic acid and alpha-linolenic acid (ALA). These dietary EFAs are found in the brain only in small quantities and it is their desaturated metabolites which are found in abundance. The first step in the metabolism of linoleic acid and ALA is 6-desaturation, a step which is rate limiting. This is why the 6-desaturated EFAs are particularly valuable in combination with cerium, caesium or indium.

The principle EFAs, of value, with their conversions, are:

3

| n-6 | n-3 |
|---|---|
| 18:2 delta-9,12(linoleic acid) | 18:3 delta-9,12,15 (alpha-linolenic acid) |
| delta-6 desaturase | |
| 18:3 delta-6,9,12(gamma-linolenic acid) | 18:4 delta-6,9,12,15 |
| elongation | |
| 20:3 delta-8,11,14(dihomo-gamma-linolenic acid) | 20:4 delta-8,11,14,17 |
| delta-5 desaturase | |
| 20:4 delta-5,8,11,14(arachidonic acid) | 20:5 delta-5,8,11,14,17 |
| elongation | |
| 22:4 delta-7,10,13,16(adrenic acid) | 22:5 delta-7,10,13,16,19 |
| delta-4 desaturase | |
| 22:5 delta-4,7,10,13,16 | 22:6 delta-4,7,10,13,16,19 |

The acids are in the natural all-cis configuration. In the n-6 series, commonly used names for the 18:2 and 18:3 (octadeca di-enoic and tri-enoic) acids are linoleic acid and gamma-linolenic acid (GLA); for the 20:3 and 20:4 (eicosa tri-enoic and tetra-enoic) acids they are dihomo-gamma-linolenic acid (DGLA) and arachidonic acid (AA); and for the 22:4 (docosatetraenoic) acid, adrenic acid. In the n-3 series only alpha-linolenic acid (ALA) (18:3) is commonly referred to by a non-systematic name but the name stearidonic acid does exist for the 18:4 n-3 acid. Initials derived from the systematic names are also used e.g. EPA for the 20:5 (eicosapentaenoic) acid, but do not serve where acids of the same chain length and degree of unsaturation exist in both series, e.g. the two 22:5 acids.

## DOSE RANGES

1 microg to 500mg of indium, caesium or cerium, preferably 100 microg to 10mg, daily, corresponding to dosage unit forms comprising such amounts/dose. Essential fatty acids 1microg to 100g, preferably 100 microg to 10g, daily, again corresponding to dosage unit forms containing such amounts/dose.

## FORMS AND SOURCES OF EFAs

When derivatives of the EFAs are used as discussed below, the amounts are calculated as the EFA.

The EFAs can be and indeed normally will be used as an assimilable, pharmacologically acceptable and physiologically equivalent derivative convertible in the body to the acid and reference to such EFAs herein (including in the claims) is to be taken as including reference to such derivatives. Identification of useful derivatives is by their having the valuable effect in the body of the acid itself, but conversion can be shown directly by gas chromatographic analysis of concentrations in blood, body fat, or other tissue by standard techniques, for example those of Pelick et al, p. 23, "Analysis of Lipids and Lipoproteins" Ed. Perkins, American Oil Chemists Society, Champaign, Illinois, U.S.A.

Convenient derivatives of EFAs include salts, amides, esters including glyceride esters and alkyl (e.g. $C_1$ to $C_4$) esters, and phospholipids.

Thus if desired, pharmaceutical compositions may be produced for use in the invention so far as the EFA is concerned by associating the natural or synthetic acid, as such or as a derivative, with an acceptable pharmaceutical vehicle. It is, however, at present convenient to incorporate the acids into compositions in the form of available oils having a high content of the acids, hence references to "oils" herein.

Fish oils are a convenient source of n-3 EFAs, examples being fish body oils, especially of oily fish such as herring, mackerel, anchovies, pilchards, menhaden, tuna and salmon, fish gut oils, fish liver oils;

oils derived from growth of certain fungi and algae; oils from animals or birds consuming fish, such as seals.

At the present time known natural oils having a high GLA acid content are few (there are no known natural sources of significant amounts of DGLA). One source of GLA currently available is the seed of Evening Primrose species such as Oenothera biennis L. and Oenothera lamarckiana, the oil extract therefrom containing GLA (about 8%) and linoleic acid (about 72%) in the form of their glycerides together with other glycerides (percentages based on total fatty acids). Other sources of GLA are Borage species such as Borago officinalis which, though current yield per acre is low, provide a richer source of gamma-linolenic acid than Oenothera oil. Recent studies on fungi and other micro-organisms which can be cultivated by fermentation promise a micro-organism source.

The oil is extracted from the seeds by one of the conventional methods of extraction such as cold pressure, screw pressure after partially cooking the seed or solvent extraction.

Fractionation of a typical sample of this oil in the form of methyl esters shows the relative proportions:

Palmitate       6.15
Stearate        1.6
Oleate          10.15
Linoleate       72.6
Gamma-Linolenate    8.9

The seed oil extracts referred to above can be used as such or can, for example, if desired, be fractionated to yield an oily composition containing the triglycerides of gamma-linolenic and linoleic as the main fatty acid components, the gamma-linolenic acid content being if desired a major proportion. Seed oil extracts appear to have a stabilising effect upon DGLA if present.

For the n-6 series acids higher than GLA, synthesis is possible though not simple, for example for DGLA. However, AA and higher acids are available from slaughterhouses, for example adrenic acid from adrenal glands. Arachidonic acid is present in substantial amounts in meat.

## PACKS

If it is not desired to have compositions comprising different active materials together, packs may be prepared comprising the materials presented for separate, or part joint and part separate administration in the appropriate relative amounts, and use of such packs is within the purview of this invention.

## DIETARY COMPOSITIONS

The invention is chiefly described in relation to methods of treatment and pharmaceutical compositions, but it will be understood that the EFAs, being in the nature of dietary supplements, could be incorporated in a dietary margarine or other foodstuff. It is also possible that indium, caesium or cerium compounds could be incorporated into foods or nutritional supplements, especially ones prepared specifically for use by patients with schizophrenia or other disorders associated with brain atrophy, and such foodstuffs are "medicaments" for the purposes of this specification.

## PHARMACEUTICAL PRESENTATION

The compositions according to the invention are conveniently in a form suitable for oral, parenteral, etc. administration in a suitable pharmaceutical vehicle, as discussed in detail for example in Williams GB-A-1,082,624 to which reference may be made, and in any case very well known generally for any particular kind of preparation. Thus, for example, tablets, capsules, ingestible liquid or powder preparations, creams and lotions for topical application or suppositories can be prepared as required. The compositions according to the present invention as their fatty acid derivatives may be particularly appropriate for topical application allowing penetration through the skin. Injectable solutions of hydrolysed Oenothera oil and fish oil may be prepared using albumin to solubilise the free acid.

It will be understood that the absolute quantity of active materials present in any dosage unit should not

5

exceed that appropriate to the rate and manner of administration to be employed but on the other hand should also desirably be adequate to allow the desired rate of administration to be achieved by a small number of doses. The rate of administration will moreover depend on the precise pharmacological action desired.

## EXAMPLES

1. Tablets containing 1mg of indium, caesium or cerium chloride, one per day, administered alone or with capsules of evening primrose oil 1000mg to 10g to persons suffering from schizophrenia or the other conditions given herein.

2. Tablets containing 2mg of indium, caesium or cerium oxalate, one per day, administered alone or with capsules of evening primrose oil 2000mg to 10g per day to persons suffering from schizophrenia or the other conditions given herein.

3. Soft gelatine capsules containing 10mg of indium, caesium or cerium gamma-linolenate, dihomo-gamma-linolenate, arachidonate, eicosapentaenoate or docosahexaenoate, one per day, administered to persons suffering from said conditions.

4. Emulsions for intravenous administration once per day, containing 5mg of indium, caesium or cerium gamma-linolenate, dihomo-gamma-linolenate, arachidonate, eicosapentaenoate or docosahexaenoate, administered once per 1 to 7 days.

5. Depot preparations in oily vehicles or with dextrans containing 100mg of one or more of the above salts of cerium, indium or caesium for intramuscular or subcutaneous injection and providing for slow release of the active materials, administered once per 1 to 12 weeks.

6. Solutions for parenteral administration, such as intravenous, intraperitoneal or into the cerebrospinal fluid containing 5mg/ml of cerium chloride, caesium chloride or indium chloride, administered once per 1 to 7 days.

7. Topical preparations of essential fatty acids in which daily doses as above are applied to the skin of persons suffering from said conditions, accompanied by indium, caesium or cerium in the same preparation or by another systemic route.

8. Topical preparations of indium, caesium or cerium gamma-linolenate, dihomo-gamma-linolenate, arachidonate, eicosapentaenoate or docosahexaenoate in which daily doses as above are applied to the skin of persons suffering from said conditions.

## Claims

1. A method of preparation of a medicament for treatment of schizophrenia or other chronic cerebral disorder in which cerebral atrophy is shown, characterised in that one or more assimilable or pharmaceutically acceptable indium caesium or cerium compounds is associated with a pharmaceutically acceptable diluent or carrier.

2. A method according to claim 1 in which the or each compound is an essential fatty acid salt, particularly of gamma-linolenic acid or higher n-6 acid, or stearidonic acid or high n-3 acid.

3. A method according to claim 1 or claim 2 in which the carrier itself comprises an essential fatty acid, particularly of gamma-linolenic acid or higher n-6 acid, or stearidonic acid or high n-3 acid.

4. A method according to claim 1, 2 or 3 in which the medicament is in dosage unit form comprising 1 mg to 500 mg of the or each said compound calculated as the metal.

5. A method according to claim 1, 2 or 3 in which the medicament is in dosage unit form comprising 1 mg to 100 g essential fatty acid calculated as free acid.

6. Indium, caesium or cerium in the form of an essential fatty salt thereof, particularly of gamma-linolenic acid or higher n-6 acid, or stearidonic acid or high n-3 acid.

7. An assimilable and pharmaceutically acceptable compound of indium, caesium or cerium for use in therapy, including therapy of schizophrenia or other chronic cerebral disorders in which cerebral atrophy is shown.

8. A compound according to claim 7, being an essential fatty acid salt, particularly of gamma- linolenic acid or higher n-6 acid, or stearidonic acid or high n-3 acid.

9. A compound according to claim 7 or claim 8, in a pharmaceutically acceptable diluent or carrier.

10. A compound according to claim 9 wherein the carrier itself comprises an essential fatty acid, particularly of gamma-linolenic acid or higher n-6 acid, or stearidonic acid or high n-3 acid.

11. A compound according to any of claims 7 to 10 has a dosage unit form composition comprising 1 mg to 500mg of the or each compound calculated as the metal.

12. A compound according to any of claims 7 to 11, has a dosage unit form composition comprising 1 mg to 100mg essential fatty acid calculated as the free acid.